# EUROPEAN PATENT SPECIFICATION

(11) **EP 4 260 844 B1**
(45) Date of publication and mention of the grant of the patent: **18.03.2026**
(21) Application number: 22167707.3
(22) Date of filing: 11.04.2022
(51) Int. Cl.: A61K 31/4709, A61K 31/496, A61K 31/505, A61K 31/546, A61K 31/57, A61K 31/573, A61K 31/58, A61K 31/7036, A61K 38/12, A61K 38/14, A61K 9/00, A61K 9/10, A61K 45/06, A61K 47/10, A61P 1/00

(54) **OPHTHALMOLOGICAL COMPOSITIONS COMPRISING POLOXAMER**
OPHTHALMOLOGISCHE ZUSAMMENSETZUNGEN MIT POLOXAMER
COMPOSITIONS OPHTALMOLOGIQUES COMPRENANT DU POLOXAMÈRE

(43) Date of publication of application: 18.10.2023
(73) Proprietor: Liegner, Jeffery T., Newton, New Jersey 07860 (US); Karolchyk, John Scott, Lake Hopatcong, New Jersey 07849 (US)
(72) Inventor: Liegner, Jeffery T., Newton, New Jersey 07860 (US); Karolchyk, John Scott, Lake Hopatcong, New Jersey 07849 (US)
(74) Representative: Lee, Nicholas John

(56) References cited:
- WO-A1-2015/012899
- US-A1- 2015 025 511

## Description

### FIELD OF THE INVENTION

The present invention relates generally to the field of ophthalmology and more specifically to ophthalmological compositions having anti-bacterial and anti-inflammatory properties, methods of preparing such compositions, and to medical uses of such compositions.

### BACKGROUND

In ophthalmological treatments and procedures, e.g., cataract surgery, pre- and post-operative eye drops are frequently used by the patients to eliminate or alleviate negative post-surgery complications such as infections, inflammation, and tissue edema. It has been reported that as many as 8% of all ocular surgery patients may suffer from infections, including the potentially catastrophic endophthalmitis, and various negative sight threatening side effects after surgery, such as inflammatory uveitis, corneal edema, and cystoid macular edema. Typically, the topical postoperative medications are prescribed for at-home use starting before and then after cataract surgery, and are typically self-administered.

These ophthalmic medication drops include anti-inflammatory and antibiotic agents. WO 2015012899 A1 describes an ophthalmic formulation.

It has been observed that certain ophthalmic formulations can exhibit precipitation after preparation, which can have potential implications on the bioavailability of the anti-inflammatory and anti-bacterial agents in the formulation.

In view of the above, there is therefore a need for a new formulation with anti-bacterial and anti-inflammatory properties for application in patients undergoing ophthalmological treatments and procedures which exhibits reduced precipitation after preparation.

### SUMMARY

The present invention is as set out in the appended claims.

Accordingly, in a first aspect of the invention there is provided a pharmaceutical composition, comprising:
(a) moxifloxacin hydrochloride at a concentration of about 0.1% w/v;
(b) triamcinolone acetonide micronized (TAC) at a concentration of about 1.5% w/v;
(c) poloxamer 407 (poly(ethylene glycol)-block-poly(propylene glycol)-block-poly(ethylene glycol)) at a concentration of about 1.2% w/v;
(d) polysorbate 80 at a concentration of about 1% w/v;
(e) edetate calcium disodium at a concentration of about 0.2% w/v;
(f) hydrochloric acid (HCl); and
(g) QS 100ml water.

In a second aspect of the invention there is provided a method for preparing a pharmaceutical composition comprising combining components (a) to (g) of the first aspect of the invention, optionally wherein the components are combined in a one-batch formulation method.

In a third aspect of the invention there is provided the composition of the first aspect of the invention for use in medicine.

In a fourth aspect of the invention there is provided the composition of the first aspect of the invention for use in a method for treating an ophthalmological disease, condition or pathology, optionally selected from sight-treating postoperative endophthalmitis and ocular inflammation.

In a fifth aspect of the invention there is provided pharmaceutical kit, comprising a sealed container containing the pharmaceutical composition of the first aspect of the invention and an instruction for the use of the composition enclosed with the container.

It has been unexpectedly discovered that the aforementioned ophthalmological compositions comprising poloxamer 407, at a concentration of 1.2% w/v, avoid precipitation for at least 21 days after preparation. It has also been discovered that such beneficial effect cannot be obtained with concentrations of poloxamer 407 below 1.2% w/v. Such compositions exhibit anti-bacterial prophylaxis and suppression of postoperative inflammation making them suitable for patients undergoing ophthalmological treatments and procedures.

### DETAILED DESCRIPTION

### A. Terms and Definitions

Unless specific definitions are provided, the nomenclatures utilized in connection with, and the laboratory procedures and techniques of analytical chemistry, synthetic organic and inorganic chemistry described herein, are those known in the art. Standard chemical symbols are used interchangeably with the full names represented by such symbols. Thus, for example, the terms "hydrogen" and "H" are understood to have identical meaning. Standard techniques may be used for chemical syntheses, chemical analyses, formulating compositions and testing them. The foregoing techniques and procedures can be generally performed according to conventional methods well known in the art.

It is to be understood that both the foregoing general description and the following detailed description are exemplary and explanatory only and are not restrictive of the invention claimed. As used herein, the use of the singular includes the plural unless specifically stated otherwise. The section headings used herein are for organizational purposes only and are not to be construed as limiting the subject matter described.

As used herein, "or" means "and/or" unless stated otherwise. Furthermore, use of the term "including" as well as other forms, such as "includes," and "included," is not limiting.

"About" as used herein means that a number referred to as "about" comprises the recited number plus or minus 1-5% of that recited number. For example, "about" 100 degrees can mean 95-105 degrees or as few as 99-101 degrees depending on the context. Whenever it appears herein, a numerical range such as "1 to 20" refers to each integer in the given range; i.e., meaning only 1, only 2, only 3, etc., up to and including only 20.

The term "pharmaceutical composition" is defined as a chemical or a biological compound or substance, or a mixture or combination of two or more such compounds or substances, intended for use in the medical diagnosis, cure, treatment, or prevention of disease or pathology.

The term "intraocular injection" refers to an injection that is administered by entering the eyeball of the patient.

The term "transzonular" refers to an injection administered through the ciliary zonule which is a series of fibers connecting the ciliary body and lens of the eye.

The term "intravitreal" refers to an injection administered through an eye of the patient, directly into the inner cavity of the eye.

The term "intraoperative" is defined as an action occurring or carried during, or in the course of, surgery.

The terms "anti-bacterial" and "antibiotic" used herein interchangeably, refer to substances or compounds that destroy bacteria and/or inhibit the growth thereof via any mechanism or route.

The term "anti-inflammatory" refers to substances or compounds that counteract or suppress inflammation via any mechanism or route.

The term "quinolone" for the purposes of this application refers to a genus of anti-bacterial compounds that are derivatives of benzopyridine and in some embodiments include fluorine atom, such as in the following structure ("fluoroquinolone"):

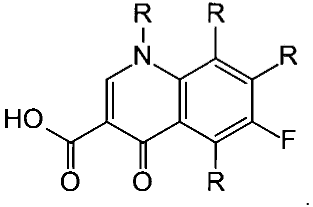

The term "corticosteroid" is defined as a compound belonging to a sub-genus of steroids that are derivatives of corticosterone, the latter having the chemical structure:

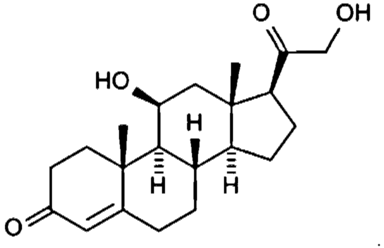

The term "salt" refers to an ionic compound which is a product of the neutralization reaction of an acid and a base.

The terms "solvate" and "hydrate" are used herein to indicate that a compound or a substance is physically or chemically associated with a solvent for "solvates" such as water (for "hydrates") .

The term "ether" refers to a chemical compound containing the structure R-O-R₁, where two organic fragments R and R₁ are connected via oxygen.

The term "ester" refers to a chemical compound containing the ester group R-O-C(O)-R₁, connecting two organic fragments R and R₁.

The terms "acetal" and "ketal" refer to a chemical compound containing the functional group R-C(R₁)(OR₂)₂, where R and R₂ are organic fragments and R₁ is hydrogen atom (for acetals), and is inclusive of "hemiacetals" where one R₂ (but not the other) is hydrogen atom; or where none of R, R₁ and R₂ is a hydrogen atom and each is an organic fragment (for ketals).

The term "carrier" refers to a substance that serves as a vehicle for improving the efficiency of delivery and the effectiveness of a pharmaceutical composition.

The term "excipient" refers to a pharmacologically inactive substance that is formulated in combination with the pharmacologically active ingredient of pharmaceutical composition and is inclusive of bulking agents, fillers, diluents and products used for facilitating drug absorption or solubility or for other pharmacokinetic considerations.

The term "therapeutically effective amount" is defined as the amount of the compound or pharmaceutical composition that will elicit the biological or medical response of a tissue, system, animal or human that is being sought by the researcher, medical doctor or other clinician.

The term "pharmaceutically acceptable" is defined as a carrier, whether diluent or excipient, that is compatible with the other ingredients of the formulation and not deleterious to the recipient thereof.

The terms "administration of a composition" or "administering a composition" is defined to include an act of providing a compound of the invention or pharmaceutical composition to the subject in need of treatment.

### B. Embodiments of the Invention

According to embodiments of the present invention, the pharmaceutical composition can be for delivery in the form of eye drops.

According to embodiments of the present invention, the pharmaceutical composition can be for delivery in the form of eye sprays.

According to embodiments of the present invention, the polysorbate 80 can be present in the pharmaceutical composition at a concentration of 1% w/v.

According to embodiments of the present invention, the poloxamer 407 can be present in the pharmaceutical composition at a concentration of 1.2%.

According to embodiments of the present invention, polysorbate 80 can be present in the pharmaceutical composition in at a concentration of 1.0% w/v and poloxamer 407 can be present in the composition at a concentration of 1.2% w/v.

According to embodiments of the present invention, the pharmaceutical composition can further comprise a therapeutically effective quantity of an antibiotic selected from the group consisting of vancomycin, teicoplanin, telavancin, decaplanin, ramoplanin, gentamicin, tobramycin, amikacin, cefuroxime, polymyxin B sulfate, trimethoprim, and a combination thereof.

According to embodiments of the present invention, the method can comprise delivery of the pharmaceutical composition to a subject by means selected from the group consisting of intravitreal injection, intraocular intracameral injection, intra-lesional injection, intra-articular injection, subconjunctival injection, sub-tenon injection, delivery via eye drops, delivery via spray and intra-canalicular delivery, optionally wherein the subject is a mammalian subject.

According to embodiments of the present invention, pharmaceutical compositions intended to prevent and/or treat inflammation and/or infections are provided. In some embodiments, the pharmaceutical compositions can be used for intraocular injections. In other embodiments the pharmaceutical compositions can be used for intra-articular or intra-lesional use.

The pharmaceutical composition of the present invention comprises moxifloxacin hydrochloride. Moxifloxacin (chemically, 1-cyclopropyl-7-[(1S,6S)-2,8-diazabicyclo-[4.3.0]non-8-yl]-6-fluoro-8-methoxy-4-oxo-quinoline-3-carboxylic acid), is available, e.g., under trade name Avelox^{®} from Bayer Healthcare Corp. of Wayne, New Jersey, and under other trade names from other suppliers such as Alcon Corp. and Bristol-Myers Squibb Co. and has the following chemical structure:

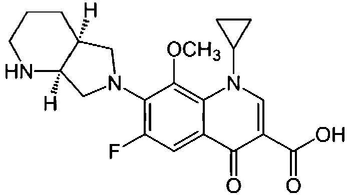

A non-limiting example of a possible alternative fluoroquinolone antibiotic that may be used in combination with moxifloxacin is gatifloxacin. In some embodiments one or several glycopeptide antibiotic(s), or a combination of some or all of them, may be optionally used as a part of the anti-bacterial agent, in combination with moxifloxacin. One example of such an acceptable additional glycopeptide antibiotic is vancomycin which can be introduced into the pharmaceutical composition at a concentration between about 1mg/mL and about 100.0 mg/mL, such as between about 5.0 mg/mL and about 50.0 mg/mL, for example, about 10.0 mg/mL. Vancomycin is available under the trade name Vancocin^{®} from Eli Lilly & Co. of Indianapolis, Indiana. Other acceptable additional glycopeptide antibiotics that may be used include teicoplanin, telavancin, decaplanin, ramoplanin, gentamicin, tobramycin, amikacin, cefuroxime, polymyxin B sulfate, and trimethoprim. The composition of the present invention comprises the corticosteroid triamcinolone acetonide (chemically, (4aS,4bR,5S,6aS,6bS,9aR,10aS,10bS)-4b-fluoro-6b-glycoloyl-5-hydroxy-4a,6a,8,8-tetramethyl-4a,4b,5,6,6a,6b,9a,10,10a,10b,11,12-dodecahydro-2H-naphtho[2',1':4,5]indeno[1,2-d][1,3]dioxol-2-one) which is a ketal derivative of triamcinolone available, e.g., under the trade name Kenalog^{®} from Bristol-Myers Squibb Co. of Princeton, New Jersey, and under other trade names from other suppliers, and having the following chemical formula:

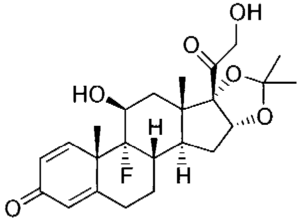

Other corticosteroids include triamcinolone diacetate, triamcinolone benetonide, triamcinolone furetonide, triamcinolone hexacetonide, betamethasone acetate, dexamethasone, fluorometholone, fluocinolone acetonide and difluoroprednisolone butyrate acetate (DFBA).

As mentioned above, the pharmaceutical composition that is the subject matter of the instant application may may further optionally include one or several pharmaceutically acceptable excipient(s) in addition to poloxamer 407 and polysorbate 80. Those having ordinary skill in the art will be able to select the suitable excipient(s). It is worth mentioning that when moxifloxacin is used in pharmaceutical formulations, it is often difficult to obtain a stable suspension of another product (e.g., a corticosteroid such as triamcinolone acetonide) that is present in the same formulation and that needs to be in a form of a stable suspension. Without being bound by any particular scientific theory, such difficulties in obtaining the stable suspension are believed to be caused by moxifloxacin's tendency to deactivate many suspending agents resulting in unacceptable coagulation, clumping and flocculation. As a result, normal delivery through a typical 27-29 gage cannula is often difficult or even impossible.

Therefore, it is desirable to select an excipient that is stable in the presence of moxifloxacin and can, therefore, be used as a solubilizing and suspending agent to ensure that the corticosteroid such as triamcinolone acetonide safely forms a stable suspension when moxifloxacin is also present in the same formulation. Numerous attempts by others to produce a stable moxifloxacin/triamcinolone acetonide pharmaceutical composition suitable for intraocular injection have not been successful.

Poloxamer 407 is used as an excipient in the pharmaceutical compositions of the present invention, for example, as a solubilizing and stabilizing agent to obtain a stable suspension of the corticosteroid triamcinolone acetonide.

The term "poloxamer" represents a class of organic structures that have certain chain structures and repeating hydrocarbon configurations. More specifically, poloxamers are nonionic triblock copolymers composed of a central hydrophobic chain of polyoxypropylene (poly(propylene oxide)), PPO, flanked by two hydrophilic chains of polyoxyethylene (poly(ethylene oxide)), PEO. Because the lengths of the polymer blocks can be customized, poloxamer is a class of molecular structures that demonstrate a wide range of chemical properties (phase behavior, solubility, reactivity, toxicity *et al*.). There are thousands of poloxamer molecules, in a wide range of molecular weights and stereochemical configurations, that have been created, tested and explored for various industrial and medical applications. Many are clinically toxic and medically unacceptable, including distinctively limited applications if applied topical versus injectable.

For the generic term "poloxamer", these copolymers are commonly named with the letter P (for poloxamer) followed by three digits: the first two digits multiplied by 100 give the approximate molecular mass of the polyoxypropylene core, and the last digit multiplied by 10 gives the percentage polyoxyethylene content (e.g. P407 = poloxamer with a polyoxypropylene molecular mass of 4000 g/mol and a 70% polyoxyethylene content). See Schmolka IR. Poloxamers in the pharmaceutical industry. In: Tarcha PJ, ed. Polymers for Controlled Drug Delivery, CRC Press, Boca Raton, FL 1991, pp 189-214.

Poloxamers sold under the Pluronics brand have a slightly different nomenclature system. In the Pluronic nomenclature, the first letter stands for solid ("F"), paste ("P") or liquid ("L"). As with the general poloxamer nomenclature, the last digit approximates the weight content of EO block in tens of weight percent. The remaining first one or two digits encode the molecular mass of the central PPO block. To decipher the code, one should multiply the corresponding number by 300 to obtain the approximate molecular mass in Da. Thus, for example, the code "Pluronic F127" defines the block copolymer, which is a solid, has a PO block of 3600 Da (12 x 300) and 70% wt. of PEO. See Appendix A of Kabanov et al., "Pluronic® block copolymers: novel functional molecules for gene therapy," Advanced Drug Delivery Reviews, 54:223-233 (2002). The Pluronic code can be converted to the general poloxamer code by multiplying the first two digits by 3. Thus, Pluronic F127 would correspond to poloxamer 367.

Poloxamer 407 (poly(ethylene glycol)-block-poly(propylene glycol)-block-poly(ethylene glycol)) is a specific non-ionic polyoxyethlene-polyoxypropylene block copolymer with the molecular weight of the polyoxypropylene portion of about 4,000 Daltons, about a 70% polyoxyethylene content, the overall molecular weight of between about 9,840 Daltons and about 14,600 Daltons and having the following chemical structure: 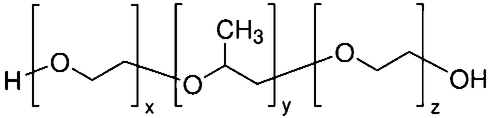 wherein the approximate block lengths can be X=101, Y=56 and Z=101. Poloxamer 407 is available from, for example, Sigma-Aldrich Corp. of St. Louis, Missouri.

The concentration of poloxamer 407 in the composition of the present invention is about 1.2% w/v and preferably 1.2% w/v.

Polysorbate 80 is used as an excipient in the pharmaceutical composition of the present invention, for example, as an emulsifier. Polysorbates are a class of emulsifiers that are oily liquids derived from ethoxylated sorbitan (a derivative of sorbitol) esterified with fatty acids. The number following the 'polysorbate' part of the name is related to the type of fatty acid associated with the polyoxyethylene sorbitan part of the molecule. Polysorbate 20 is polyoxyethylene (20) sorbitan monolaurate. Polysorbate 40 is polyoxyethylene (20) sorbitan monopalmitate. Polysorbate 60 is polyoxyethylene (20) sorbitan monostearate. Polysorbate 80 is polyoxyethylene (20) sorbitan monooleate.

The concentration of polysorbate 80 in the composition of the present invention is about 1.0% w/v and preferably 1.0% w/v.

The other excipients and carriers that are used in the pharmaceutical compositions include, edetate calcium disodium (EDTA, a chelating agent), hydrochloric acid (the pH adjuster) and sterile water.

Disclosed herein is a pharmaceutical composition comprising (or preferably consisting of) difluoroprednisolone butyrate acetate (DFBA) (preferably 0.01% w/v), moxifloxacin hydrochloride (preferably 0.1% w/v), poloxamer 407 (preferably 0.06 to 0.2% w/v), polysorbate 80 (preferably 0.08 to 0.12% w/v), edetate calcium disodium (preferably 0.2% w/v), hydrochloric acid (preferably to adjust pH of the composition in the range of from 6.5 to 7.6, more preferably to adjust pH of the composition to 7.0) and water (preferably QS to 100ml).

Disclosed herein is a pharmaceutical composition comprising (or preferably consisting of) triamcinolone acetonide micronized (TAC) (preferably 1.5% w/v), moxifloxacin hydrochloride (preferably 0.1% w/v), sodium carboxymethylcellulose (preferably 0.3 to 0.4% w/v), polysorbate 80 (preferably 0.00001 to 0.00003% w/v), edetate calcium disodium (preferably 0.2% w/v), sodium chloride (preferably 0.6% w/v), hydrochloric acid (preferably to adjust pH of the composition in the range of from 6.5 to 7.6, more preferably to adjust pH of the composition to 7.0) and water (preferably QS to 100ml).

Disclosed herein is a pharmaceutical composition comprising (or preferably consisting of) triamcinolone acetonide micronized (TAC) (preferably 1.5% w/v), moxifloxacin hydrochloride (preferably 0.1% w/v), sodium carboxymethylcellulose (preferably 0.3 to 0.4% w/v), polysorbate 80 (preferably 0.01 to 0.03% w/v), edetate calcium disodium (preferably 0.2% w/v), sodium chloride (preferably 0.6% w/v), hydrochloric acid (preferably to adjust pH of the composition in the range of from 6.5 to 7.6, more preferably to adjust pH of the composition to 7.0) and water (preferably QS to 100ml).

The pharmaceutical compositions described herein may be used in methods for treating an ophthalmological disease, condition or pathology in a mammalian subject in need of such treatment, which comprise delivery to the subject a pharmaceutical composition described herein, wherein the method of delivery is selected from the group consisting of intravitreal injection, intraocular intracameral injection, intra-lesional injection, intra-articular injection, subconjunctival injection, sub-tenon injection, delivery via eye drops, delivery via spray and intra-canalicular delivery, to treat the ophthalmological disease, condition or pathology thereby.

According to further embodiments, methods for fabricating the above-described pharmaceutical compositions are provided. A one-batch formulation method may be used, where the components of the pharmaceutical formulation can be combined in single container; the components may be added to the container simultaneously or consecutively.

In one exemplary, non-limiting procedure, a quantity of an anti-bacterial agent such as moxifloxacin may be placed into a mixing container followed by adding a quantity of sterile water and hydrochloric acid to obtain a slightly acidic mixture (e.g., having pH of about 6.5) which is stirred until a clear solution is obtained. In case of moxifloxacin/HCl system, the solution is stable, allowing the formulation to remain closed system thus preventing contamination and the loss of sterility.

Next, a quantity of corticosteroid such as micronized triamcinolone acetonide, a quantity of poloxamer 407, a quantity of edetate calcium disodium and a quantity of polysorbate 80 may be all added to be combined in the same container with the already prepared moxifloxacin/HCl solution and stirred together (e.g., by spinning) for a period of time, e.g., about 6 hours, until a homogenous suspension has been obtained. The resulting suspension may then be transferred into single dose vials, capped, sealed, autoclaved and shaken until cool. Finally, a complete testing for sterility and the presence of endotoxin may be performed on the product according to commonly used methods known to those having ordinary skill in the art.

Pharmaceutical compositions prepared as described above can be used to prevent complications that may arise after ophthalmic surgical operations and procedure. For example, the formulations can be used during any intraocular surgery, such as cataract surgery, planned vitrectomy or glaucoma procedures, to prevent or at least substantially reduce the risk of post-surgery complications, such as the development of endophthalmitis or cystoid macular edema (CME), without having the patient use pre- or post-operative topical ophthalmic drops. Individuals with evidence of endophthalmitis from prior surgical procedures or traumatic ocular penetration will benefit from concurrent injection of these formulations to sterilize infection and reduce damaging inflammation.

Pharmaceutical formulations described herein can be delivered via intraocular intravitreal injection which can be transzonular, or, if desired not transzonular. Intraocular intravitreal injection of this formulation, whether done via transzonular or via direct pars plana (trans-scleral) injection, delivers potent broad spectrum antibiotics directly into the suppurative tissue without requiring the urgent compounding of multiple individual medications or multiple individual injections into the eye.

Typically, a pharmaceutical composition described above will be intraocularly administered to a mammalian subject (e.g., humans, cats, dogs, other pets, domestic, wild or farm animals) in need of emergent, urgent or planned ophthalmic surgery treatment. The effect achieved by such use of pharmaceutical composition described above may last up to four weeks. The composition is to be injected intravitreally and trans-zonularly using methods and techniques known to those having ordinary skilled in the art of ophthalmology. In some embodiments, the injection can be intraoperative.

Typically, the delivery through a typical 27 gauge cannula can be employed utilizing a 1 mL TB syringe, with attention to re-suspending the formulation using momentary flicks and shake just prior to injection. The medicinal volume (i.e., dosage) required of this formulation varies based on the type of intraocular procedure, the degree of postoperative inflammation induced or anticipated, the risk assessment for postoperative infection, and anatomic considerations regarding the available volume for the injection being added to a closed intraocular space.

It is worth mentioning that while intracameral (that is, anterior chamber) injections are within the scope of the instant invention such injections instead of posterior chamber (intravitreal) injection may not be satisfactory in some cases, as the suspension clogs the trabecular meshwork and aggravates intraocular drainage, resulting in an intraocular pressure rise postoperative. This is avoided with intravitreal injection, in addition to retaining the formulation components into the protein matrix of the vitreous of a greater duration. Anterior chamber wash out occurs over hours (antibiotic in solution) and days (steroid in suspension), while intravitreal injection is retained for weeks.

The pharmaceutical compositions described herein may be intravitreally transzonularly injected into a mammalian subject as a part of the process of treatment of a variety of ophthalmological diseases, conditions or pathologies associated with intraocular surgery, such as cataracts, retinal and glaucoma disease.

In alternative embodiments, if desired or necessary the formulations may preferably be delivered in the form of eye drops or eye sprays. In alternative embodiments, if desired or necessary the formulations may be delivered via subconjunctival injection, intraocular intracameral injection, sub-tenon injection, intra-articular injection or intra-lesional injection, particularly, in, but not limited to, some cases when necessary to deliver additional medication when local ocular inflammation and extra-ocular infection need suppression. Intravitreal delivery of steroid has historically been used to treat clinically significant cystoid macular edema (CME); the application of this formulation into the vitreous during routine intraocular procedures brings more aggressive prophylaxis against CME occurrence. Additionally, the suspension of this formulation is useful for staining vitreous during planned and unplanned vitrectomies, improving visualization of this otherwise transparent intraocular tissue, improving vitrectomy outcomes and reducing complications resulting from inadequate or tractional vitreous removal. In still further embodiments, there is also envisioned intra-canalicular delivery, i.e., delivery via a lacrimal canaliculus implant.

In some further alternative embodiments, instead of delivering the above-described compositions comprising both anti-bacterial and anti-inflammatory agents, consecutive injections may be used instead, if desired. For example, triamcinolone may be injected first, immediately followed by the injection of moxifloxacin or vice versa.

It will be understood by those having ordinary skill in the art that the specific dose level and frequency of dosage for any particular patient may be varied and will depend upon a variety of factors including the activity of the specific compound employed, the metabolic stability and length of action of that compound, the age, body weight, general health, gender, diet, and the severity of the particular ophthalmological condition being treated.

In additional embodiments, pharmaceutical kits are provided. The kit includes a sealed container approved for the storage of pharmaceutical compositions, the container containing one of the above-described pharmaceutical compositions. An instruction for the use of the composition and the information about the composition are to be included in the kit.

The following examples are provided to further elucidate the advantages and features of the present invention, but are not intended to limit the scope of the invention. The examples are for the illustrative purposes only. USP pharmaceutical grade products were used in preparing the formulations described below.

### C. Examples

### Example 1. Preparing a Pharmaceutical Composition

A pharmaceutical composition was prepared as described below. The following products were used in the amounts and concentrations specified:
(a) triamcinolone acetonide micronized (TAC), at a concentration of about 1.5% w/v;
(b) moxifloxacin hydrochloride, at a concentration of about 0.1% w/v;
(c) polysorbate 80, at a concentration of about 1% w/v;
(d) edetate calcium disodium, at a concentration of about 0.2% w/v;
(e) poloxamer 407, at a concentration of about 1.2% w/v (variable in Example 3 below);
(f) hydrochloric acid, to adjust pH; and
(g) about 100.0 mL of sterile water for injection.

### Example 1A. Preparatory Method 1

Moxifloxacin hydrochloride was placed into a de-pyrogenated beaker with a spin bar. Sterile water for injection was added to about 1/3 of the volume of the beaker. While spinning, moxifloxacin was dissolved by adding hydrochloric acid until a clear solution having the final pH of about 6.5 was obtained.

The solution was combined with micronized triamcinolone acetonide, poloxamer 407, edetate calcium disodium and polysorbate 80 and allowed to spin for about 6 hours until a hydrated and homogenous suspension was obtained.

The suspension was transferred into de-pyrogenated, single dose vials (2mL size), capped and sealed, followed by autoclaving and shaking the vials until cool. Complete sterility and endotoxin testing was performed by an outside laboratory to ensure safety.

The formulation prepared as described above was tested for stability after 6 months of storage. After this period of storage no loss of potency was observed (as measured by HPLC); the formulation was visually stable at room temperature and readily re-suspended with gentle shaking with no increase of particle size or flocculation.

### Example 1B Preparatory Method 2

Moxifloxacin was dissolved in a beaker in 1/3 of the water, and HCl was added until pH = 6 and the resulting composition was mixed until clear.

The other ingredients in Example 1 were added to the composition, the QS was adjusted to the final volume of 100ml and the resulting composition was homogenized at 5,000 RPM for 30 minutes.

The composition was then transferred to autoclave bottle, autoclaved at 121°C with 15 psi for 30 minutes and then shaken until cool.

### Example 2. Characterization of the Pharmaceutical Compositions

In Example 3 below, each formulation prepared according to Examples 1A/1B was subjected to testing, which included pH stability, viscosity, turbidity, flocculation, precipitation (aggregation by contact adhesion), product and drug stability after sterilization, syringeability (delivery through ophthalmic cannula), and each batch's expectation for clinical efficacy. Such tests included observation of sedimentation, flocculation, aggregation, syringeability, and chromatographic chemical testing of the compositions at one week, one month, three months. pH was measured with an Horiba Ion Selective Electrode device. Viscosity was measured using a rheometer from Anton-Parr. Turbidity was visually assessed and measured using a Multi Angle Light Scattering (MALS) detector by Malvern. Flocculation and Particulate Size was measured using the Multi Angle Light Scattering (MALS) detector by Malvern. Precipitation (Layering Out of Solution) was visually assessed over time, noting aggregation at bottom of test tube. Product Stability was assessed with various modalities: drug integrity, chromatography, syringeability, etc. Syringe and Cannula Delivery was assessed by syringe delivery through various gauge cannulas. Drug and Product Integrity after sterilization was assessed with various modalities: drug integrity, chromatography, syringeability, etc. Clinical Efficacy was assessed by physician based on constellation of findings, procedural functionality, and predictive performance with patients.

### Example 3. - Effect of Different Poloxamer 407 Concentrations

In this Example, compositions were prepared in accordance with Examples 1A/1B, with variable poloxamer 407 concentrations, and characterized in accordance with Example 2.

Experiments were conducted testing formulations of triamcinolone and moxifloxacin in the relative amounts (Tri-Moxi) recited in Example 1 with various concentrations of the preferred poloxamer 407, using 1% w/v polysorbate 80. The summary of the results are shown in Table 1 below.

Conclusion: The presence of poloxamer 407 at concentrations below 2.2% w/v is required to have a clinically effective formulation. Poloxamer 407 at 2.2%, 5% and 10% w/v concentrations, are clinically FAILED formulations for this ophthalmic application. Poloxamer 407 concentrations in the range of from 0.8% to 2.0% w/v were functionally capable formulations for clinical applications required of this novel drug combination. Unexpectantly, poloxamer 407 concentrations in the range of from 1.2% to 2.0% w/v exhibited no precipitation for at least 21 days compared with minimal precipitation for poloxamer 407 concentrations below 1.2%.

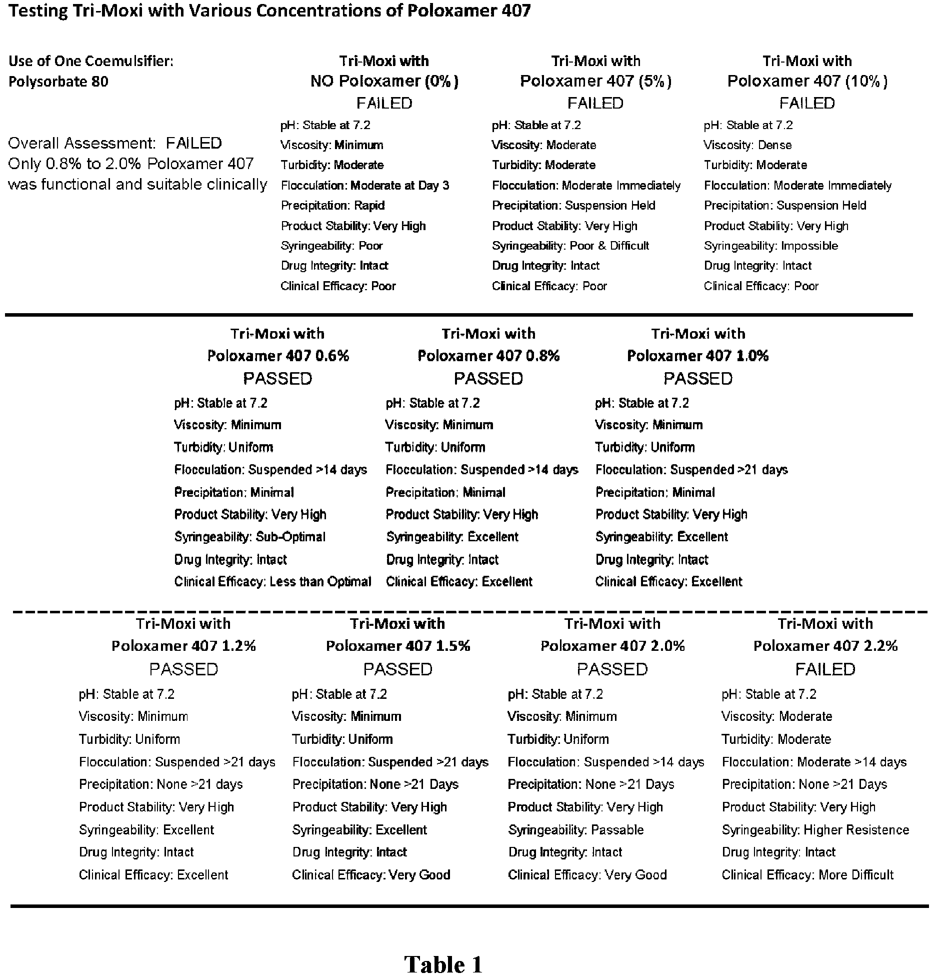

These experiments show that the incorporation of poloxamer 407 into the formulation at a concentration of 1.2 - 2.0% w/v has surprising and unexpected beneficial precipitation properties. The beneficial precipitation properties enable a preservative-free therapeutic drug combination (antibiotic and corticosteroid) with excellent storage properties that is suitable for injection into the intraocular space as well as for topical application, providing protection and prophylaxis against sight-treating postoperative endophthalmitis and ocular inflammation.

### Example 4. Preparing a Pharmaceutical Composition Containing Vancomycin

A pharmaceutical composition was prepared as described in Example 1A/1B, *supra.* The composition was autoclaved and sonicated for about 60 minutes and about 96 mL of the composition were combined with about 4 mL of vancomycin at a concentration of about 250 mg/mL. The pH of the mixture was adjusted to about 6.0-6.5 using hydrochloric acid. The product was then transferred into vials (at about 1 mL plus 5 drops per vial) and frozen. The product has kept its stability and potency for at least six months.

### Example 5. Using a Pharmaceutical Composition

A pharmaceutical composition fabricated as described in Example 1, *supra,* was administered to about 1,600 patients. To each, it was introduced using intravitreal transzonular injection. The injection was intraoperative. Only a very few patients, at the rate of about only 1 in 4,000, have developed any infection or suffered from other side effects that required further treatment, which is a substantial improvement over a typical rate of about 8% for the patients that did not receive the injection.

## Claims

1. A pharmaceutical composition, comprising:
(a) moxifloxacin hydrochloride at a concentration of about 0.1% w/v;
(b) triamcinolone acetonide micronized (TAC) at a concentration of about 1.5% w/v;
(c) poloxamer 407 (poly(ethylene glycol)-block-poly(propylene glycol)-block-poly(ethylene glycol)) at a concentration of about 1.2% w/v;
(d) polysorbate 80 at a concentration of about 1% w/v;
(e) edetate calcium disodium at a concentration of about 0.2% w/v;
(f) hydrochloric acid (HCl); and
(g) QS 100ml water.

2. The pharmaceutical composition of claim 1, wherein the composition is for delivery in the form of eye drops or eye sprays.

3. The pharmaceutical composition of claim 1 or 2, further comprising a therapeutically effective quantity of an antibiotic selected from the group consisting of vancomycin, teicoplanin, telavancin, decaplanin, ramoplanin, gentamicin, tobramycin, amikacin, cefuroxime, polymyxin B sulfate, trimethoprim, and a combination thereof.

4. A method for preparing a pharmaceutical composition comprising combining components (a) to (g) of any preceding claim.

5. The method of claim 4, wherein the components are combined in a one-batch formulation method.

6. The composition of any one of claims 1 to 3 for use in medicine.

7. The composition of any one of claims 1 to 3 for use in a method for treating an ophthalmological disease, condition or pathology, optionally selected from sight-treating postoperative endophthalmitis and ocular inflammation.

8. The composition for use of claim 7, wherein the method comprises delivery of the composition to a subject by means selected from the group consisting of intravitreal injection, intraocular intracameral injection, intra-lesional injection, intra-articular injection, subconjunctival injection, sub-tenon injection, delivery via eye drops, delivery via spray and intra-canalicular delivery, optionally wherein the subject is a mammalian subject.

9. A pharmaceutical kit, comprising a sealed container containing the pharmaceutical composition of any one of claims 1 to 3 and an instruction for the use of the composition enclosed with the container.

## Patentansprüche

1. Pharmazeutische Zusammensetzung, umfassend:
(a) Moxifloxacin-Hydrochlorid in einer Konzentration von etwa 0,1 % (Gew./Vol.);
(b) mikronisiertes Triamcinolonacetonid (TAC) in einer Konzentration von etwa 1,5 % (Gew./Vol.);
(c) Poloxamer 407 (Poly(ethylenglykol)-Block-Poly(propylenglykol)-Block-Poly(ethylenglykol)) in einer Konzentration von etwa 1,2 % (Gew./Vol.);
(d) Polysorbat 80 in einer Konzentration von etwa 1 % (Gew./Vol.);
(e) EDTA-Calcium-Dinatrium in einer Konzentration von etwa 0,2 % (Gew./Vol.);
(f) Salzsäure (HCl); und
(g) qs 100 ml Wasser.

2. Pharmazeutische Zusammensetzung nach Anspruch 1, wobei die Zusammensetzung zur Zuführung in Form von Augentropfen oder Augensprays vorgesehen ist.

3. Pharmazeutische Zusammensetzung nach Anspruch 1 oder 2, ferner umfassend eine therapeutisch wirksame Menge eines Antibiotikums, das aus der Gruppe bestehend aus Vancomycin, Teicoplanin, Telavancin, Decaplanin, Ramoplanin, Gentamicin, Tobramycin, Amikacin, Cefuroxim, Polymyxin-B-sulfat, Trimethoprim und einer Kombination davon ausgewählt ist.

4. Verfahren zur Herstellung einer pharmazeutischen Zusammensetzung, umfassend Kombinieren der Komponenten (a) bis (g) nach einem vorhergehenden Anspruch.

5. Verfahren nach Anspruch 4, wobei die Komponenten in einem Ein-Ansatz-Formulierungsverfahren kombiniert werden.

6. Zusammensetzung nach einem der Ansprüche 1 bis 3 zur medizinischen Verwendung.

7. Zusammensetzung nach einem der Ansprüche 1 bis 3 zur Verwendung bei einem Verfahren zur Behandlung einer ophthalmologischen Erkrankung bzw. eines ophthalmologischen Leidens oder Krankheitsbilds, das gegebenenfalls aus Sichtbehandlung von postoperativer Endophthalmitis und Augenentzündung ausgewählt ist.

8. Zusammensetzung zur Verwendung nach Anspruch 7, wobei das Verfahren Zuführung der Zusammensetzung an ein Individuum mit Mitteln umfasst, die aus der Gruppe bestehend aus intravitrealer Injektion, intraokularer intrakameraler Injektion, intraläsionaler Injektion, intraartikulärer Injektion, subkonjunktivaler Injektion, Subtenon-Injektion, Zuführung über Augentropfen, Zuführung über Spray und intrakanalikulärer Zuführung ausgewählt sind, gegebenenfalls wobei es sich bei dem Individuum um ein Säugerindividuum handelt.

9. Pharmazeutisches Kit, umfassend einen verschlossenen Behälter, der die pharmazeutische Zusammensetzung nach einem der Ansprüche 1 bis 3 enthält, und eine dem Behälter beiliegende Anweisung zur Verwendung der Zusammensetzung.

## Revendications

1. Composition pharmaceutique, comprenant :
(a) chlorhydrate de moxifloxacine à une concentration d'environ 0,1 % p/v ;
(b) acétonide de triamcinolone micronisé (TAC) à une concentration d'environ 1,5 % p/v ;
(c) poloxamère 407 (poly(éthylène glycol)-bloc-poly(propylène glycol)-bloc-poly(éthylène glycol)) à une concentration d'environ 1,2 % p/v ;
(d) polysorbate 80 à une concentration d'environ 1 % p/v ;
(e) édétate de calcium disodique à une concentration d'environ 0,2 % p/v ;
(f) acide chlorhydrique (HCl) ; et
(g) qsp 100 ml d'eau.

2. Composition pharmaceutique selon la revendication 1, dans laquelle la composition est destinée à être administrée sous la forme de gouttes oculaires ou de sprays oculaires.

3. Composition pharmaceutique selon la revendication 1 ou 2, comprenant en outre une quantité thérapeutiquement efficace d'un antibiotique choisi dans le groupe constitué par vancomycine, teicoplanine, télavancine, décaplanine, ramoplanine, gentamicine, tobramycine, amikacine, céfuroxime, sulfate de polymyxine B, triméthoprime et une combinaison de ceux-ci.

4. Procédé de préparation d'une composition pharmaceutique comprenant la combinaison des composants (a) à (g) selon l'une quelconque des revendications précédentes.

5. Procédé selon la revendication 4, dans lequel les composants sont combinés dans un procédé de formulation en un lot.

6. Composition selon l'une quelconque des revendications 1 à 3 pour une utilisation en médecine.

7. Composition selon l'une quelconque des revendications 1 à 3 pour une utilisation dans un procédé de traitement d'une maladie, d'un état ou d'une pathologie ophtalmologique, éventuellement choisi(e) parmi l'endophtalmie postopératoire de traitement de la vue et l'inflammation oculaire.

8. Composition pour utilisation selon la revendication 7, dans laquelle le procédé comprend l'administration de la composition à un sujet par des moyens choisis dans le groupe constitué par une injection intravitréenne, une injection intraoculaire intracamérale, une injection intralésionnelle, une injection intra-articulaire, une injection sous-conjonctivale, une injection sous-ténonienne, une administration par gouttes oculaires, une administration par pulvérisation et une administration intracanaliculaire, éventuellement le sujet étant un sujet mammifère.

9. Kit pharmaceutique, comprenant un récipient scellé contenant la composition pharmaceutique selon l'une quelconque des revendications 1 à 3 et une instruction pour l'utilisation de la composition jointe au récipient.
